# EUROPEAN PATENT APPLICATION

(11) **EP 4 062 932 A2**
(43) Date of publication of application: **28.09.2022**
(21) Application number: 20891298.0
(22) Date of filing: 16.11.2020
(51) Int. Cl.: A61K 39/39, A61K 31/353, A61K 39/00

(54) **NOVEL VACCINE IMMUNE ADJUVANT COMPOSITION CONTAINING BAVACHIN**

(30) Priority: 22.11.2019 KR 20190151536
(71) Applicant: Korea Institute of Oriental Medicine, Daejeon 34054 (KR); Korea Research Institute of Chemical Technology, Daejeon 34114 (KR)
(72) Inventor: JIN, Young Hee, Daejeon 34054 (KR); KWON, Sun Oh, Daejeon 34054 (KR); KIM, Dong Eon, Daejeon 34054 (KR); MIN, Jung Sun, Daejeon 34054 (KR); JANG, Min Seong, Daejeon 34114 (KR)
(74) Representative: Ström & Gulliksson AB
(86) International application number: PCT/KR2020/016066
(87) International publication number: WO 2021/101187

(57) **Abstract**

Provided are a vaccine adjuvant composition including, as an active ingredient, bavachin capable of improving antibody titer and enhancing cellular immunity and humoral immunity when administered together with an antigen, a vaccine preparation including the bavachin and an antigen, a method of promoting an immune response, the method including the step of administering the vaccine adjuvant composition to a subject together with a vaccine composition or before and after administration of the vaccine composition, and a vaccine adjuvant composition for promoting an immune response, the vaccine adjuvant composition including the bavachin. Since the vaccine adjuvant composition of the present invention includes bavachin isolated from an extract of *Psoralea corylifolia,* of which safety has been secured, it may exhibit safety and may enhance both the humoral and cellular immune responses as well as the titer of antibodies generated by antigens, and thus the vaccine adjuvant composition may be widely used in the development of effective vaccine preparations.

## Description

### BACKGROUND OF THE INVENTION

### 1. Field of the Invention

The present invention relates to a vaccine adjuvant composition including bavachin as an active ingredient, and more particularly, the present invention relates to a vaccine adjuvant composition including, as an active ingredient, bavachin capable of improving antibody titer and enhancing cellular immunity and humoral immunity when administered together with an antigen, a vaccine preparation including the bavachin and an antigen, a method of promoting an immune response, the method including the step of administering the vaccine adjuvant composition to a subject together with a vaccine composition or before and after administration of the vaccine composition, and a vaccine adjuvant composition for promoting an immune response, the vaccine adjuvant composition including the bavachin.

### 2. Description of the Related Art

A vaccine is a medicine used to generate an immune response to an antigen for the purpose of defending against a pathogenic infection. Vaccines that have recently been developed mainly use recombinant proteins as antigens. Recombinant protein vaccines have fewer side effects and are safer than killed vaccines or live attenuated vaccines, but they are poorly immunogenic. Thus, a vaccine adjuvant is used together to generate sufficient immunity to defend against infection.

Vaccine adjuvants are largely divided into three types according to the mechanism of action: antigen carriers, immune enhancers, and those that stimulate an immune response, and at the same time, act as a matrix for antigens. When vaccine adjuvants are effectively used, various effects of (1) increasing the immunogenicity of recombinant antigens, (2) reducing the antigen dose or the number of immunizations, and (3) improving immunogenicity in infants and the elderly with weak immunity may be obtained. However, despite the usefulness of these vaccine adjuvants, there are still not many vaccine adjuvants licensed for use in human vaccines, because many factors should be considered to be selected as vaccine adjuvants. The conditions for ideal vaccine adjuvants are that they have excellent safety and tolerability with a simple preparation method thereof and low cost. In addition, they should have a long half-life in the body and be biodegradable, and immune responses to the vaccine adjuvants themselves should not be induced. Among these, the safety issue of vaccine adjuvants is the most important.

Currently, vaccine adjuvants approved for use in vaccines in Europe and the United States include aluminum salts, MF59, AS03, AS04, *etc.* Aluminum salts are mainly used in the form of Al(OH)₃ or AlPO₄, and it was generally thought that aluminum salts exhibit an immune enhancing effect by adsorbing protein antigens and releasing them slowly. However, aluminum salts have recently been found to activate dendritic cells and to promote secretion of cytokines such as interleukin (IL)-1β and IL-18. Aluminum salts are widely used in several vaccines and are thought to be very safe, but it is assumed that they cause allergic reactions and are neurotoxic. In addition, aluminum salts strongly induce an antibody-mediated humoral immune response, but hardly induce a cellular immune response, and are disadvantageous in that cryopreservation is impossible.

In view of this technical background, the present inventors have made intensive efforts to develop more effective vaccine adjuvants, and as a result, they found that bavachin may be used as an active ingredient for vaccine adjuvants, thereby completing the present invention.

### SUMMARY OF THE INVENTION

An object of the present invention is to provide a vaccine adjuvant composition including bavachin as an active ingredient.

Another object of the present invention is to provide a vaccine preparation including the bavachin and an antigen.

Still another object of the present invention is to provide a method of promoting an immune response, the method including the step of administering the vaccine adjuvant composition to a subject together with a vaccine composition or before and after administration of the vaccine composition.

Still another object of the present invention is to provide a vaccine adjuvant composition for promoting an immune response, the vaccine adjuvant composition including the bavachin.

### BRIEF DESCRIPTION OF THE DRAWINGS

FIG. 1 shows graphs showing results of comparing OVA-specific antibody titers according to use of bavachin;
FIG. 2 shows graphs showing results of analyzing isotypes of immunoglobulins in the sera according to use of bavachin;
FIG. 3 shows graphs showing results of measuring cell proliferation ability of splenocytes and lymph node cells according to use of bavachin;
FIG. 4 shows graphs showing results of comparing levels of cytokines (IFN-y, IL-2, IL-4, IL-6, IL-10, and TNF) secreted from splenocytes and lymph node cells according to use of bavachin;
FIG. 5 shows graphs showing results of comparing the numbers of splenocytes and lymph node cells according to use of bavachin;
FIG. 6 shows graphs showing results of comparing percentages of CD4 cells and CD8 cells in splenocytes according to use of bavachin; and
FIG. 7 shows graphs showing results of comparing percentages of effector T cells in lymph node cells according to use of bavachin.

### DETAILED DESCRIPTION OF THE PREFERRED EMBODIMENTS

To achieve the above objects, one aspect of the preset invention provides a vaccine adjuvant composition including bavachin.

Further, another aspect of the present invention provides use of the vaccine adjuvant composition including bavachin.

As used herein, the term "bavachin" refers to a kind of compounds included in an extract of *Psoralea corylifolia,* and refers to a compound having a structure of the following Formula 1:

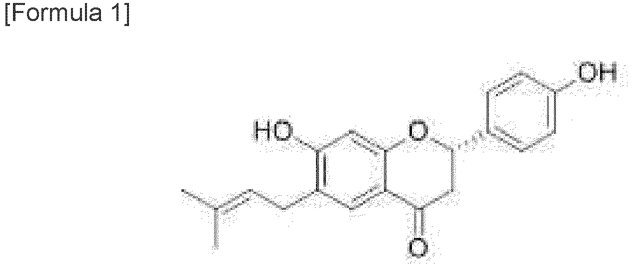

As used herein, the term "vaccine adjuvant" refers to a pharmaceutical or immunological preparation administered for the purpose of enhancing immune responses of vaccines.

When administered together with antigens, the vaccine adjuvant composition provided in the present invention may improve antibody titer and may enhance cellular immunity and humoral immunity at the same time.

In particular, since the vaccine adjuvant includes, as an active ingredient, bavachin derived from an extract of *Psoralea corylifolia,* of which safety has been secured, it may be safely used *in vivo.*

The vaccine adjuvant composition may further include a pharmaceutically acceptable adjuvant, excipient, or diluent, in addition to the carrier.

As used herein, the "pharmaceutically acceptable" composition is a composition that is physiologically acceptable and does not cause an allergic reaction such as gastrointestinal disorder, or dizziness, or a similar reaction thereof when administered to humans. Examples of the carrier, excipient, and diluent may include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. In addition, fillers, anti-agglomeration agents, lubricants, wetting agents, fragrances, emulsifiers, preservatives, *etc.* may be further included.

The vaccine composition may further include a commonly used vaccine adjuvant, in addition to the vaccine adjuvant. Examples of the vaccine adjuvant may include aluminum hydroxide, aluminum phosphate, alum (potassium aluminum sulfate), MF59, virosome, AS04 (a mixture of aluminum hydroxide and monophosphoryl lipid A (MPL)), AS03 (a mixture of DL-α-tocopherol, squalene, and an emulsifier polysorbate 80), CpG, Flagellin, Poly I:C, AS01, AS02, ISCOMs, ISCOMMATRIX, *etc.*

In addition, the vaccine adjuvant composition may be formulated using a method known in the art to allow rapid release, or sustained or delayed release of an active ingredient when administered to a mammal. Formulations include powders, granules, tablets, emulsions, syrups, aerosols, soft or hard gelatin capsules, sterile injectable solutions, and sterile powders.

The vaccine adjuvant composition of the present invention may be administered via various routes including, for example, oral and parenteral routes, e.g., suppository, transdermal, intravenous, intraperitoneal, intramuscular, intralesional, intranasal, and intraspinal routes, and may be administered using an implantable device for sustained or continuous or repeated release. Administrations may be carried out once or several times a day within a desired range, and the administration period is not particularly limited.

The vaccine adjuvant composition of the present invention may be administered by way of a general systemic or local administration method, e.g., intramuscular injection or intravenous injection, and for example, it may be injected by means of an electroporator. The electroporator may include an electroporator for injecting commercially available DNA drugs into the body, e.g., Glinporator (manufactured by IGEA, Italy), CUY21 EDIT (manufactured by JCBIO, Korea), SP-4a (manufactured by Supertech, Switzerland), OrbiJector (manufactured by SLVAXiGEN, Korea), *etc.*

With regard to the administration route, the vaccine adjuvant composition of the present invention may be administered via any common route as long as it is able to reach a tissue of interest. Such administration route may be, but is not limited to, parenteral routes, for example, intraperitoneal, intravenous, intramuscular, subcutaneous, and intrasynovial routes.

The vaccine adjuvant composition of the present invention may be formulated in a suitable form together with a commonly used pharmaceutically acceptable carriers. Pharmaceutically acceptable carriers may include, for example, water, suitable oils, saline solution, carriers for parenteral administration such as aqueous glucose and glycols, *etc.,* and the vaccine adjuvant composition may further include a stabilizer and a preservative. Examples of suitable stabilizers are antioxidants such as sodium hydrogen sulfite, sodium sulfite, and ascorbic acid. Examples of suitable preservatives are benzalkonium chloride, methyl- or propyl-paraben, and chlorobutanol.

As needed, the vaccine adjuvant composition of the present invention may appropriately include a suspending agent, a solubilizer, a stabilizer, an isotonic agent, a preservative, an adsorption inhibitor, a surfactant, a diluent, an excipient, a pH adjuster, an analgesic agent, a buffering agent, an antioxidant, *etc.,* according to the administration method or formulations. Pharmaceutically acceptable carriers and preparations suitable for the present invention, including those exemplified above, are described in detail in the literature (*Remington's Pharmaceutical Sciences,* recent edition).

When the vaccine adjuvant composition of the present invention is administered to a subject, the dosage varies depending on many factors, including the subject's height, body surface area, age, a particular compound to be administered, sex, time and route of administration, general health conditions, and other drugs to be administered simultaneously. For example, the vaccine adjuvant composition may be administered in an amount of 100 ng/kg (body weight) to 10 mg/kg (body weight), for another example, in an amount of 1 µg/kg (body weight) to 500 µg/kg (body weight), and for still another example, in an amount of 5 µg/kg (body weight) to 50 µg/kg (body weight).

The vaccine composition of the present invention may be administered in a pharmaceutically effective amount.

As used herein, the term "pharmaceutically effective amount" refers to an amount sufficient for the treatment of diseases at a reasonable benefit/risk ratio applicable to medical treatment, and the level of the effective dose may be determined according to factors including the kind of subject, severity of illness, age, sex, drug activity, drug sensitivity, administration time, administration route and excretion rate, period of treatment, drug(s) to be used simultaneously, and other factors well known in the medical field. The vaccine adjuvant composition of the present invention may be administered, for example, at a dose of 0.1 mg/kg to 1 g/kg, and for another example, at a dose of 1 mg/kg to 500 mg/kg. Meanwhile, the administration dose may be appropriately adjusted according to a patient's age, sex, and health conditions.

Still another aspect of the present invention provides a vaccine preparation including the bavachin and an antigen.

The antigen is not particularly limited as long as it is able to induce an immune response *in vivo.* For example, the antigen may be a virus antigen, a cancer antigen, or a combination thereof, as another example, the antigen may be a virus antigen derived from various viruses such as Epstein-Barr virus (EBV), hepatitis A virus (HAV), hepatitis B virus (HBV), hepatitis C virus (HCV), hepatitis D virus (HDV), hepatitis E virus (HEV), Hantaan virus, cytomegalovirus (CMV), human immunodeficiency virus (HIV), influenza virus, human papilloma virus (HPV), poliovirus, Ebola virus, rotavirus, dengue virus, West Nile virus, yellow fever virus, adenovirus, Japanese encephalitis virus, BK virus, smallpox virus, Zika virus, severe fever with thrombocytopenia syndrome virus (SFTS virus), herpes simplex virus (HSV), *etc.,* and as still another example, the antigen may be a human papilloma virus (HPV)-derived antigen, a carcinoembryonic antigen, a prostate-specific antigen (PSA), prostate specific membrane antigen (PSMA), Her2/neu, MUC-1, BCR/ABL, alpha-fetoprotein (AFP), an Epstein-Barr virus (EBV)-derived antigen, a human hepatitis B virus (HBV)-derived antigen, a human hepatitis C virus (HCV)-derived antigen, a cancer antigen such as cancer antigen-125 (CA-125), cancer antigen-72-4 (CA-72-4), cancer antigen-15-3 (CA-15-3), cancer antigen-19-9 (CA-19-9), *etc.*

Still another aspect of the present invention provides a method of promoting an immune response, the method including the step of administering the vaccine adjuvant composition and an antigen to a subject in need of immune activity against the antigen.

In the method, the vaccine adjuvant composition may be administered together with an antigen inducing an immune response or together with a vaccine composition including the antigen, or may be administered to a subject before or after administration of the vaccine composition.

Still another aspect of the present invention provides a vaccine adjuvant composition for promoting an immune response, the vaccine adjuvant composition including the bavachin.

Still another aspect of the present invention provides use of the vaccine adjuvant composition including the bavachin in promoting an immune response.

As described above, when the bavachin is administered together with an antigen, it may improve antibody titer and may enhance cellular immunity and humoral immunity at the same time, and therefore, the composition including the bavachin may be used as a vaccine adjuvant for promoting an immune response.

Hereinafter, the present invention will be described in more detail with reference to exemplary embodiments. However, these exemplary embodiments are for illustrative purposes only, and the scope of the present invention is not intended to be limited by these exemplary embodiments.

### Example 1: Preparation of Vaccine Adjuvant Composition including Bavachin

100 µg of bavachin was dissolved in 0.5% CMC to prepare a vaccine adjuvant composition.

### Example 2: Mouse Immunization

C57/BL6 mice were immunized with a mixture of OVA (10 µg) and bavachin (10 µg) or a mixture of OVA (10 µg) and bavachin (100 µg) by subcutaneous injection once every 1, 3, and 4 weeks. In this regard, those injected with OVA (10 µg) alone was used as a control.

### Example 3: Analysis of Antibody Titer in Mouse Blood

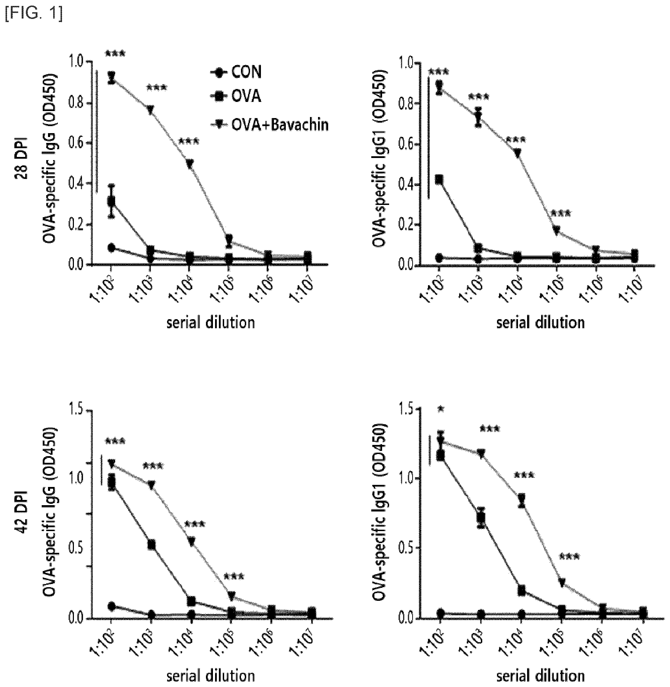

At 28 days and 42 days after the first immunization in Example 2, mouse sera were collected, and the titers of antigen-specific IgG antibodies in the sera were measured by way of ELISA (FIG. 1). At this time, in the ELISA method, an OVA-coated plate was blocked, and then sera of a control group and an experimental group were incubated at various serial dilution rates. Thereafter, horseradish peroxidase-conjugated mouse-IgG and IgG1 antibodies were added, and a substrate was added and allowed to react, followed by measurement with an ELISA reader.

FIG. 1 shows graphs showing results of comparing the OVA-specific antibody titers according to use of bavachin.

As shown in FIG. 1, it was confirmed that the OVA-specific antibody titers in the mice injected with bavachin together with OVA significantly increased, as compared with OVA antigen-specific antibody titers in the mice injected with OVA alone.

### Example 4: Analysis of Immune Induction by Serum Immunoglobulin Isotyping Assay

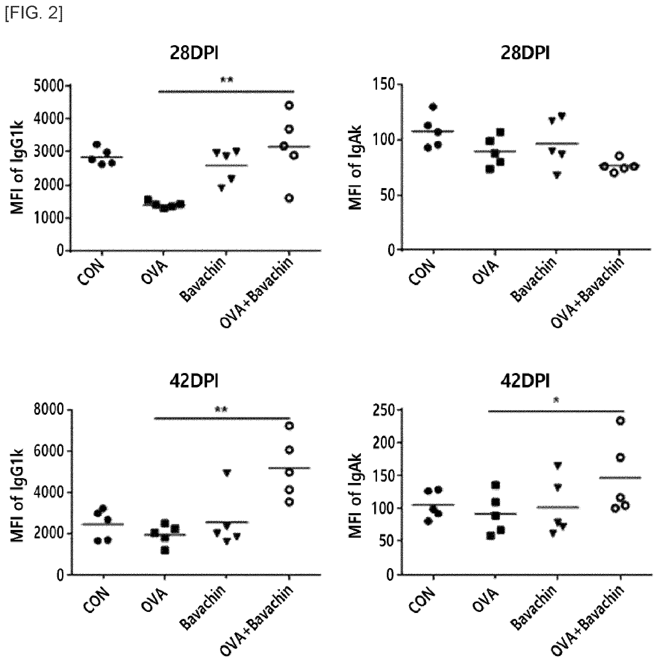

Antibody subclasses in the mouse sera collected in Example 3 were stained with CBA (Cytometric Bead Array) isotyping kit and analyzed by FACS (FIG. 2).

FIG. 2 shows graphs showing results of analyzing isotypes of immunoglobulins in the sera according to use of bavachin.

As shown in FIG. 2, it was confirmed that the sera of the mice injected with bavachin together with OVA included relatively high levels of IgG1 and IgA immunoglobulins, as compared with the sera of the mice injected with OVA alone.

Therefore, it was found that subclasses of IgG1 and IgA antibodies were increased when the bavachin was co-injected.

### Example 5: Antigen-Specific Cellular Immune Response Analysis

As shown in the results of Example 4, bavachin of the present invention was confirmed to improve humoral immunity. Therefore, it was attempted to confirm whether bavachin also affects cellular immunity.

To this end, the effects of bavachin on antigen-specific T cell proliferation and cytokine profile were investigated in the lymph nodes and spleens of mice.

Briefly, at 28 days after the first immunization, lymph nodes and spleens were removed from 5 mice per the treated mouse group (0.5% CMC (CON), OVA, Bavachin 100 µg, OVA+Bavachin), and ground to isolate cells from the tissue capsule. The spleens were subjected to hemolysis by treatment with an RBC lysis buffer, and splenocytes were isolated.

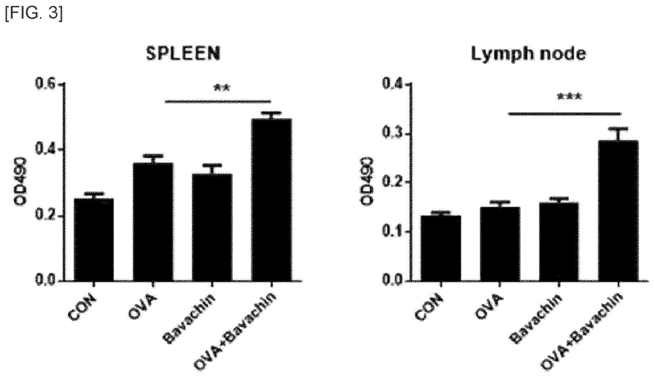

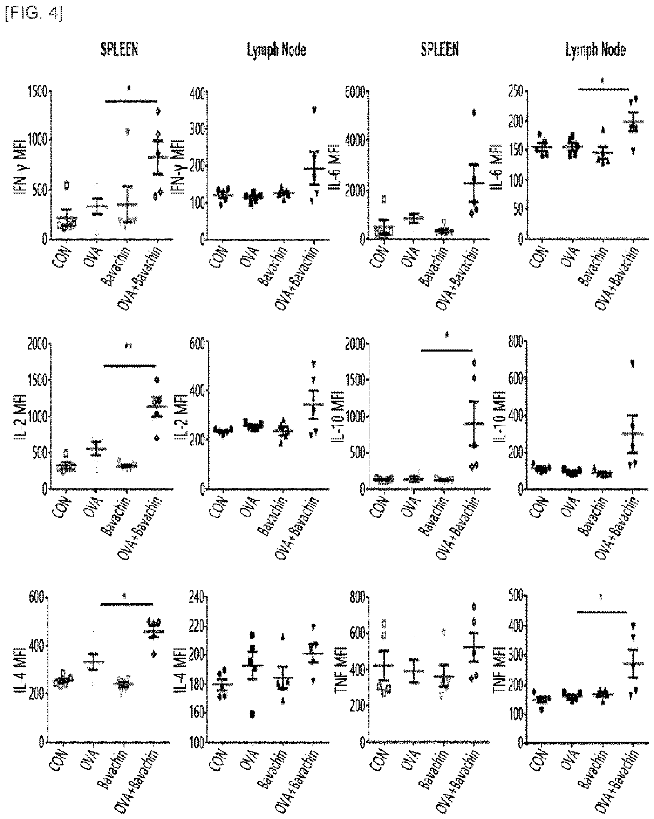

The isolated splenocytes and lymph node cells were dispensed in a 96-well-plate at a density of 1×10⁶ cells/well, followed by treated with OVA for 48 hours. Thereafter, cell proliferation was measured by MTS (FIG. 3), and cytokines (IFN-y, IL-2, IL-4, IL-6, IL-10, and TNF) secreted from the cells were measured by CBA (FIG. 4).

FIG. 3 shows graphs showing results of measuring cell proliferation ability of the splenocytes and lymph node cells according to use of bavachin.

As shown in FIG. 3, it was confirmed that the splenocytes and lymph node cells of the mice injected with bavachin together with OVA exhibited relatively excellent cell proliferation ability, as compared with the splenocytes and lymph node cells of the mice injected with OVA alone.

FIG. 4 shows graphs showing results of comparing levels of cytokines (IFN-y, IL-2, IL-4, IL-6, IL-10, and TNF) secreted from the splenocytes and lymph node cells according to use of bavachin.

As shown in FIG. 4, it was confirmed that all cytokines secreted from the splenocytes and lymph node cells of the mice injected with bavachin together with OVA were relatively high levels, regardless of the type of cytokines, as compared with those secreted from the splenocytes and lymph node cells of the mice injected with OVA alone.

The results of FIGS. 3 and 4 indicate that co-injection of bavachin increases antigen-specific T cell proliferation and cytokine secretion to improve both Th1- and Th2-type cellular immune responses.

### Example 6: Analysis of Effects on Immune Cells and Effector T Cells

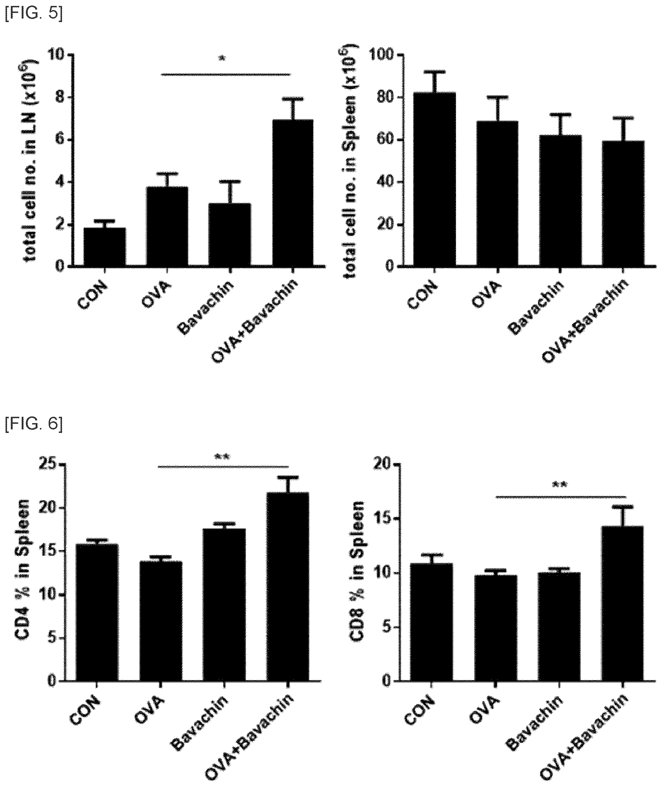

The total numbers of the splenocytes and lymph node cells obtained in Example 5 were counted (FIG. 5).

FIG. 5 shows graphs showing results of comparing the numbers of splenocytes and lymph node cells according to use of bavachin.

As shown in FIG. 5, it was confirmed that the number of lymph node cells of the mice injected with bavachin together with OVA showed relatively high levels, as compared with the number of lymph node cells of the mice injected with OVA alone.

In contrast, it was confirmed that the number of splenocytes of the mice injected with bavachin together with OVA showed no significant difference, as compared with the number of splenocytes of the mice injected with OVA alone.

Therefore, percentages of CD4 cells and CD8 cells were compared by performing FACS analysis of the splenocytes (FIG. 6).

FIG. 6 shows graphs showing results of comparing percentages of CD4 cells and CD8 cells in the splenocytes according to use of bavachin.

As shown in FIG. 6, it was confirmed that the percentages of CD4 cells and CD8 cells in the splenocytes of the mice injected with bavachin together with OVA showed relatively high levels, as compared with the percentages of CD4 cells and CD8 cells in the splenocytes of the mice injected with OVA alone.

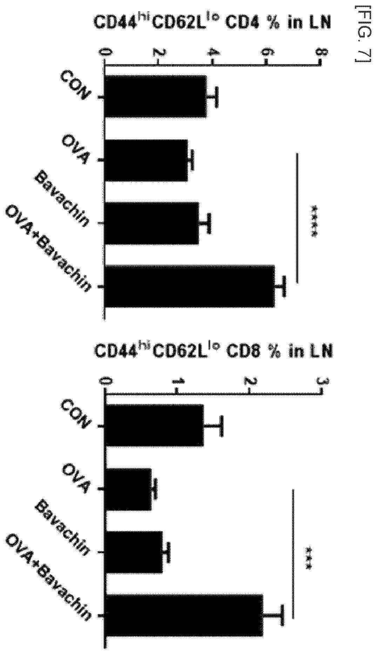

Further, percentages of CD44⁺CD62L⁻CD4 cells and CD44⁺CD62L⁻CD8 cells, which are effector T cells, were compared by performing FACS analysis of the lymph node cells (FIG. 7).

FIG. 7 shows graphs showing results of comparing percentages of the effector T cells in the lymph node cells according to use of bavachin.

As shown in FIG. 7, it was confirmed that the percentages of the effector T cells in the lymph node cells of the mice injected with bavachin together with OVA showed relatively high levels, as compared with the percentages of the effector T cells in the lymph node cells of the mice injected with OVA alone.

The results of FIGS. 5 to 7 also indicate that co-injection of bavachin improves cellular immune responses.

Taking together the results of Examples 3 to 6, when the bavachin provided in the present invention and the antigen are co-injected into the body, the titers of produced antibodies are increased to improve humoral immune responses and both Th1- and Th2-type cellular immune responses, and accordingly, the bavachin may be used as an effective vaccine adjuvant.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

### Effect of the invention

Since a vaccine adjuvant composition of the present invention includes bavachin isolated from an extract of *Psoralea corylifolia,* of which safety has been secured, it may exhibit safety and may enhance both the humoral and cellular immune responses as well as the titer of antibodies generated by antigens, and thus the vaccine adjuvant composition may be widely used in the development of effective vaccine preparations.

## Claims

1. A vaccine adjuvant composition comprising bavachin.

2. The vaccine adjuvant composition of claim 1, wherein the bavachin increases cellular immunity and humoral immunity at the same time.

3. A vaccine preparation comprising an antigen and bavachin.

4. The vaccine preparation of claim 3, wherein the antigen is an antigen selected from the group consisting of virus antigens, cancer antigens, and combinations thereof.

5. The vaccine preparation of claim 4, wherein the virus antigen is derived from a virus selected from the group consisting of Epstein-Barr virus (EBV), hepatitis A virus (HAV), hepatitis B virus (HBV), hepatitis C virus (HCV), hepatitis D virus (HDV), hepatitis E virus (HEV), Hantaan virus, cytomegalovirus (CMV), human immunodeficiency virus (HIV), influenza virus, human papilloma virus (HPV), poliovirus, Ebola virus, rotavirus, dengue virus, West Nile virus, yellow fever virus, adenovirus, Japanese encephalitis virus, BK virus, smallpox virus, Zika virus, severe fever with thrombocytopenia syndrome virus (SFTS virus), and herpes simplex virus (HSV).

6. The vaccine preparation of claim 4, wherein the cancer antigen is an antigen selected from the group consisting of a human papilloma virus (HPV)-derived antigen, a carcinoembryonic antigen, a prostate-specific antigen (PSA), prostate specific membrane antigen (PSMA), Her2/neu, MUC-1, BCR/ABL, alpha-fetoprotein (AFP), an Epstein-Barr virus (EBV)-derived antigen, a human hepatitis B virus (HBV)-derived antigen, a human hepatitis C virus (HCV)-derived antigen, cancer antigen-125 (CA-125), cancer antigen-72-4 (CA-72-4), cancer antigen-15-3 (CA-15-3), cancer antigen-19-9 (CA-19-9), and combinations thereof.

7. A method of promoting an immune response, the method comprising the step of administering an antigen and bavachin to a subject in need of immune activity against the antigen.

8. A vaccine adjuvant composition for promoting an immune response, the vaccine adjuvant composition comprising bavachin.
